# NEW EUROPEAN PATENT SPECIFICATION

(11) **EP 1 438 040 B2**
(45) Date of publication and mention of the opposition decision: **16.10.2019**
(45) Mention of the grant of the patent: 26.09.2007
(21) Application number: 02777314.2
(22) Date of filing: 16.10.2002
(51) Int. Cl.: A61K 31/365, A61K 9/28, A61P 37/06

(54) **PHARMACEUTICAL COMPOSITIONS COMPRISING MYCOPHENOLIC ACID OR MYCOPHENOLATE SALT**
PHARMAZEUTISCHE ZUSAMMENSETZUNGEN ENTHALTEND MYCOPHENOLSÄURE ODER EIN MYCOPHENOLAT SALZ
COMPOSITIONS PHARMACEUTIQUES COMPRENANT DE L'ACIDE MYCOPHENOLIQUE OU UN SEL MYCOPHENOLATE

(30) Priority: 17.10.2001 GB 0124953
(43) Date of publication of application: 21.07.2004
(73) Proprietor: Novartis AG, 4056 Basel (CH); Novartis Pharma AG, 1230 Vienna (AT)
(72) Inventor: DEDERICHS, Jürgen, 79395 Neuenburg (DE); RIGASSI, Thomas, CH-4106 Therwil (CH)
(74) Representative: Marshall, Cameron John
(86) International application number: PCT/EP2002/011589
(87) International publication number: WO 2003/032978

(56) References cited:
- WO-A-94/12184
- WO-A-94/26266
- WO-A-97/38689
- GB-A- 1 157 100
- GB-A- 1 203 328
- US-A- 5 688 529
- R. Schmouder et al: Pharmacokinetics of ERLO80A: A new enteric coated formulation of mycophenolic acid - sodium, AST Eighteenth Annual Meeting, 1999.

## Description

Mycophenolic acid, also referred to herein as MPA, a natural product of complex structure and particular sensitivity, was first isolated in 1896 and has been disclosed to have anti-tumor, anti-viral, immunosuppressive, anti-psoriatic, anti-inflammatory, and anti-cancer activity over 15 years ago. Attempts have been made to increase the bioavailability of MPA by making high molecular weight derivatives such as the morpholinomethylester of MPA, also known as mycophenolate mofetil which is being commercially used as an immunosuppressant for the treatment or prevention of organ or tissue transplant rejection. WO97/38689 and USP 6,025,391 describe a pharmaceutical composition comprising a mycophenolate salt, the composition being adapted to release the mycophenolate salt in the upper part of the intestinal tract. Capsules as representative unit dosage forms are disclosed. We have now found that these compositions are effective and tolerated for immunosuppressive indications in clinical trials.

Despite MPA being known since 1896, however, there still exists a need for commercially acceptable mycophenolic acid or mycophenolate salt dosage forms for oral administration with good patient convenience and acceptance.

GB 1203328 discloses pharmaceutical compositions comprising mycophenolic acid or a salt thereof and an inert non-toxic pharmaceutical diluent or carrier. The tablets described in this document comprise about 68 % mycophenolic acid disodium salt in combination with mercaptopurin.

WO9412184 discloses pharmaceutical compositions comprising mycophenolic acid, mycophenolate mofetil or a pharmaceutical salt or derivative thereof. In particular capsules comprising about 57 and 66 % mycophenolic acid are described.

US-A-5 688 529 discloses high dose, dry granulations and power blends and aqueous oral suspensions of mycophenolate mofetil or mycophenolic acid, containing between 7.5 and 30% mycophenolic acid.

WO 9426266 discloses high dose formulations containing mycophenolate mofetil, mycophenolic acid and ranolazine. This document describes formulations containing about 90 to 100% by weight of mycophenolic acid, in particular hard gelatine capsules comprising 90% by weight mycophenolic acid. In accordance with the present invention it has now surprisingly been found that particularly suitable pharmaceutical compositions comprising mycophenolic acid or mycophenolate salt having particularly interesting bioavailability characteristics, being well-tolerated, stable, and convenient to administer, are obtainable when the compositions are formulated as oral solid dosage forms, preferably in the form of tablets.

Difficulties in the formulation of oral solid dosage forms, e.g. tablets, comprising mycophenolic acid or mycophenolate salt, can be for example the low bulk density of the drug substance, resulting in e.g. low mechanical stability of the solid dosage form and/or unfavourable size particularly when a high amount of excipients or additives is added to improve the mechanical stability properties. Tablets with inferior mechanical properties are liable to crumble, edge chipping or break. These difficulties are even greater when it is desirable to use an oral solid dosage form with a high drug loading. Moreover, for certain groups of patients, oral administration of large tablets is either undesirable or impractical.

It has now been found that pharmaceutically acceptable oral solid dosage forms, e.g. in the form of tablets, of mycophenolic acid or mycophenolate salt are obtainable in a favourable small and mechanically stable form with a high drug loading. Oral dosage forms being particularly convenient to administer and stable, may be obtained, e.g. by preparation of tablets by compression methods. The present invention provides the process according to claim 1. Preferred embodiments are given in dependent claims 2 to 5 and the following description.

Accordingly, in one aspect the process provides an enteric coated tablet comprising a pharmacologically effective amount of mycophenolic acid or mycophenolate salt, wherein the mycophenolic acid or mycophenolate salt is present in an amount of from 20 % to 80 %, e.g. at least 35, 40, 45, 50 or 55 % to e.g 60, 65, 70, 75, 80 % or e.g 35 to 55 % by weight, preferably more than 55 % by weight based on the total weight of the solid dosage form (total solid dosage form weight being e.g the core with any coating). In particular the amount of mycophenolic acid or mycophenolate salt may be from 45 to 80 % by weight, e.g. 50 to 65 % by weight based on the total weight of the solid dosage form.

More particularly, the process provides said tablet comprising
(a) a pharmacologically effective amount of mycophenolic acid or mycophenolate salt, and
(b) pharmaceutically acceptable additives suitable for the preparation of tablets by compression methods
wherein the mycophenolic acid or mycophenolate salt is present in an amount of from 20 % to 80 %, e.g. at least 35, 40, 45, 50 or 55 % to e.g 60, 65, 70, 75, 80 % or e.g 35 to 55 % by weight, preferably more than 55 % by weight based on the total weight of the tablet (total tablet weight being e.g the core with any coating). In particular the amount of mycophenolic acid or mycophenolate salt may be from 45 to 80 % by weight, e.g. 50 to 65 % by weight based on the total weight of the tablet.

The term "pharmacologically effective amount", as used herein, is understood to mean the amount of active agent which halts or reduces the progress of the condition to be treated or which otherwise completely or partly cures or acts palliatively on the condition. Such an amount can be routinely determined by routine experimentation.

The tablets produced in accordance with the invention are, despite the high drug loading of at least 20 % by weight based on the total weight of the tablet, small, and, therefore, convenient to administer. Furthermore, said tablets were found to be stable, e.g. during storage, handling or packaging, effective and well-tolerated. Furthermore, the tablets have improved mechanical properties; e.g. when uncoated tablets are scored, they are easy to divide to produce e.g. half doses.

In addition, the tablets obtained are stable both to the production process and during storage, e.g. for 2 years or even 3 years in conventional packaging, e.g. sealed aluminium blister packs. Less than about 5%, e.g. 2 or less of mycophenolic acid or mycophenolate salt may degrade during this time as determined in conventional, e.g. stress, tests.

In another embodiment this invention provides a tablet comprising from 50 mg to 500 mg mycophenolic acid or mycophenolate salt, e.g. of from 100 mg to 500 mg mycophenolic acid or mycophenolate salt, preferably from 180 to 360 mg mycophenolic acid or from 190 to 385 mg mycophenolate salt.

When mycophenolate salt is used, cationic salts of MPA, e.g. alkali metal salts, especially the sodium salt, alkaline earth metal salts, an ammonium salt or a salt with an organic base may be used. According to the present invention, preferably the mono-sodium salt may be used. This may be obtained in crystalline form by recrystallization, e.g. from acetone/ethanol if necessary with water; m.p. 189 - 191°C.

In another aspect, the mycophenolate sodium salt is in crystalline form.

In accordance with the present invention it has surprisingly been found that it is especially advantageous to use the MPA or mycophenolate salt in the form of its anhydrate. Preferably the tablet according to the invention contains less than 5%, more preferably less than 2%, e.g. down to 0.1 or 0.3% MPA or mycophenolate salt in form of its hydrate.

Accordingly, the present invention provides a process for preparing said tablet comprising a pharmacologically effective amount of mycophenolic acid or mycophenolate salt in substantially anhydrous form. The term "in substantially anhydrous form", as used herein, is understood to mean "in an amount of 95%, preferably 98% anhydrous form".

In another aspect of the present invention, said tablet contains from 50 mg to 500 mg, preferably 100 mg to 500 mg, of crystalline mycophenolate mono sodium salt in anhydrous form.

Following pharmaceutically acceptable additives may be present in the tablets, e.g.
(1.1) one or more fillers, e.g. lactose, e.g. anhydrous lactose;
(1.2) one or more disintegrants, e.g. maize starch, Crospovidone®, or carboxymethylcellulose (CMC)-Ca;
(1.3) one or more binders, e.g. polyvinypyrrolidone, e.g. commercially as Povidone® K30;
(1.4) one or more glidants, e.g. colloidal silicon dioxide, e.g. commercially available as Aerosil® 200;
(1.5) one or more lubricants, e.g. magnesium stearate.

Reference is made to the extensive literature on the subject for these and other excipients and procedures mentioned herein, see in particular Handbook of Pharmaceutical Excipients, Second Edition, edited by Ainley Wade and Paul J. Weller, American Pharmaceutical Association, Washington, USA and Pharmaceutical Press, London; and Lexikon der Hilfsstoffe für Pharmazie, Kosmetik and angrenzende Gebiete edited by H.P. Fiedler, 4th Edition, Editio Cantor, Aulendorf and earlier editions which are incorporated herein by reference.

As fillers or diluents (1.1) we contemplate in particular sugar e.g. confectioner's sugar, compressible sugar, dextrates, dextrin, dextrose, lactose, e.g. lactose anhydrous, mannitol, microcrystalline cellulose, in particular having a density of about 0.45/cm³, e.g. commercially available as Avicel®, for example from FMC Corp., powdered cellulose, starches, e.g. maize starch, calcium phosphates, e.g. dibasic calcium phosphate dihydrate, sorbitol, sucrose and talc. Preferably lactose anhydrous may be used.

As disintegrants (1.2) maize starch, e.g. pre-gelatinized maize starch; sodium starch glycolate; croscarmellose sodium; CMC-Ca; CMC-NA; crosslinked PVP, e.g. commercially available as Crospovidone®, Polyplasdone®, from the ISP company, or Kollidon® XL; alginic acid; sodium alginate; or guar gum may be used. Preferably maize starch; crosslinked PVP, e.g. Crospovidone®; crosslinked CMC; or croscarmellose sodium, e.g. commercially available as Ac-di-sol® from FMC Corp., may be used. In particular a mixture of maize starch and crosslinked PVP may be used, e.g. in a weight ratio of from 1:1 to 1:5.

As binders (1.3) are contemplated particularly starches, e.g. potato starch, wheat starch, corn starch; microcrystalline cellulose, e.g. products known as Avicel®, Filtrak®, Heweten® or Pharmacel®; hydroxypropyl cellulose; hydroxyethyl cellulose; hydroxypropylmethyl cellulose; e.g. hydroxypropyl cellulose having a hydroxypropyl content of 5 to 16 % by weight and a Mw of from 80 000 to 1 150 000, more particularly 140 000 to 850 000; or polyvinylpyrrolidone, e.g. commercially available as Povidone® K30 from BASF. Preferably Povidone® K30 may be used.

Examples of glidants (1.4) include e.g. colloidal silica, e.g. colloidal anhydrous silica, e.g. Aerosil® 200, magnesium trisilicate, powdered cellulose, starch, talc or tribasic calcium phosphate. Preferably Aerosil® 200 may be used.

Examples of lubricants (1.5) include e.g. Mg, Al or Ca stearate, PEG 4000 - 8000, hydrogenated castor oil, glyceryl monostearate or talc. Preferably magnesium stearate may be used.

One or more of these additives can be selected and used having regard to the particular desired properties of the tablet by routine experimentation.

The amount of each type of additive employed, e.g. filler or diluent, disintegrant, binder glidant or lubricant, may be readily ascertained using procedures conventional in the art. Thus for example, the amount of filler or diluent (1.1) may vary within a range of from 5 to 40% by weight, e.g. 10 to 20% by weight;
the amount of disintegrant (1.2) may vary within a range of from 2 to 20% by weight, e.g. 10 to 15% by weight;
the amount of binder (1.3) may vary within a range of from about 1 to 45% by weight, e.g. 2 to 30% by weight, in particular 5 to 10% by weight;
the amount of glidant (1.4) may vary within ranges of from 0.1 to 10% by weight, in particular 0.1 to 5% by weight, e.g. 0.5 to 3% or 2 to 4 % by weight;
the amount of lubricant (1.5) may vary within a range of from 0.1 to 5.0% by weight, e.g. 0.5 to 2% by weight;
based on the total weight of the tablet.

It will be appreciated that any given excipient may serve more than one function e.g. as filler or diluent, disintegrant, binder glidant, and/or lubricant. The upper range of binder is preferably used in case of matrix tablets.

Preferably the tablet prepared in accordance with the invention contains as active ingredient only MPA or mycophenolate salt.

It is a characteristic of said tablet that despite the high content of MPA or mycophenolate salt, it contains only a relatively small amount of additives. This advantageously results in a mechanically stable tablet having a small size. The total amount of additives in a given unit dosage may be 65% or less by weight based on the total weight of the tablet, more particularly 50% or less. Preferably the additive content is in the range of 35 to 55% by weight, more particularly 45 to 55% by weight, e.g. 38 to 43% by weight.

The absolute amounts of each additive and the amounts relative to other additives is similarly dependent on the desired properties of the tablet and may also be chosen by routine experimentation. For example, the tablet may be chosen to exhibit accelerated and/or delayed release of the MPA or mycophenolate salt with or without quantitative control of the release of active agent. Preferably the tablet is chosen to exhibit delayed release of the mycophenolate salt, e.g. the mycophenolate mono-sodium salt.

Thus, where accelerated release is desired, e.g. about 90% release within 10 minutes, more particularly five minutes, a disintegrant such as crosslinked polyvinyl pyrrolidone, for example those products known as Polyplasdone®XL or Kollidon®CL, in particular having a molecular weight in excess of 1 000 000, more particularly having a particle size distribution of less than 400 microns or less than 74 microns, or reactive additives (effervescent mixtures) that effect rapid disintegration of the tablet in the presence of water, for example so-called effervescent tablets that contain an acid in solid form, typically citric acid, which acts in water on a base containing chemically combined carbon dioxide, for example sodium hydrogen carbonate or sodium carbonate, and releases carbon dioxide, may be used.

Whereas if delayed release is desired one can employ pellet coating technology, wax matrix systems, polymer matrix tablets or polymer coatings conventional in the art. Preferably a coating technology may be used.

Quantitative control of the release of the MPA or mycophenolate salt can be achieved by conventional techniques known in the art. Such dosage forms are known as oral osmotic systems (OROS), coated tablets, matrix tablets, press-coated tablets, multilayer tablets and the like. In a tablet according to the present invention, preferred additives are Mg stearate, anhydrous colloidal silica, maize starch, polyvinylpyrrolidone, crosslinked polyvinylpyrrolidone and anhydrous lactose. The amounts of additive employed will depend upon how much MPA or mycophenolate salt is to be used. The stearate, e.g. Mg stearate is preferably employed in amounts of 0.1 to 5.0% by weight, e.g. 0.5% to 2.0% by weight. The silica is preferably employed in an amount of from 0.1 to 10% by weight, e.g. 0.5% to 5.0% by weight.

The tablet produced in accordance with the present invention is enteric coated.

The term "enteric coating", as used herein, comprises any pharmaceutically acceptable coating preventing the release of the active agent in the stomach and sufficiently disintegrating in the intestinal tract, preferably in the upper part of the intestinal tract (by contact with approximately neutral or alkaline intestine juices) to allow the resorption of the active agent through the walls of the intestinal tract. In vitro tests for determining whether or not a coating is classified as an enteric coating have been published in the pharmacopoeia of various countries.

More specifically, the term "enteric coating", as used herein, refers to a coating which remains intact for at least 2 hours, in contact with artificial gastric juices such as HCl of pH-1 at 36 to 38° C and preferably thereafter disintegrates within 30 minutes in artificial intestinal juices such as a KH₂PO₄ buffered solution of pH 6.8.

The thickness of the coating may vary and depends inter alia on its permeability in water and acids. A typical coating may be 20 to 80 mg, e.g. 30 to 70 mg, e.g. 65 mg on a 685 mg uncoloured tablet.

In general satisfactory results are obtained with a coating of 50-200 microns, preferably 75-150 microns thickness. The coating is suitably selected from macromolecular polymers. Suitable polymers are listed in e.g. L. Lachman et al. The Theory and Practice of Industrial Pharmacy, 3rd Ed, 1986, p. 365 - 373, H. Sucker et al, Pharmazeutische Technologie, Thieme, 1991, p. 355 - 359, Hagers Handbuch der pharmazeutischen Praxis, 4th Ed. Vol. 7, pages 739 to 742 and 766 to 778, (Springer Verlag, 1971) and Remington's Pharmaceutical Sciences, 13th Ed., pages 1689 to 1691 (Mack Publ., Co., 1970) and comprise e.g. cellulose ester derivatives, cellulose ethers, acrylic resins such as methylacrylate copolymers, copolymers of maleic acid and phthalic acid derivatives, shellac, hydroxypropylmethylcellulose acetate succinate, or polyvinylacetate phthalate.

The preferred films are made from cellulose acetate phthalate and trimellitate; methacrylic acid copolymers, e.g. copolymers derived from methacrylic acid and esters thereof, containing at least 40% methacrylic acid; and especially hydroxypropyl methylcellulose phthalate, e.g. as available under the name hypromellose phthalate or hydroxypropyl methylcellulose phthalate HP50 from e.g. Shin-Etsu.

Polymethacrylates include those of molecular weight above 100,000 daltons based on, e.g. methacrylic acid and methyl or ethyl methacrylate in a ratio of about 1:1. Typical products include Eudragit L, e.g. L 100-55, or Eudragit S marketed by Röhm GmbH, Darmstadt, Germany. A mixture adapted for release of the active agent in the upper part of the intestinal tract, e.g. Eudragit L and S, may also be used. Eudragit L is preferred.

Typical cellulose acetate phthalates have an acetyl content of 17-26% and a phthalate content of from 30-40% with a viscosity of ca. 45-90cP. An example of an appropriate cellulose acetate phthalate is the marketed product CAP (Eastman Kodak, Rochester N.Y., USA).

Typical cellulose acetate trimellitates have an acetyl content of 17-26%, a trimellityl content from 25-35% with a viscosity of ca. 15-20 cS. An example of an appropriate cellulose acetate trimellitate is the marketed product CAT (Eastman Kodak Company, USA).

Hydroxypropylmethyl cellulose phthalates, typically have a molecular weight of from 20,000 to 100,000 daltons e.g. 80,000 to 130,000 daltons, e.g. a hydroxypropyl content of from 5 to 10%, a methoxy content of from 18 to 24% and a phthalyl content from 21 to 35%.

Examples of suitable hydroxypropyl methylcellulose phthalates (HPMCP) are the marketed products having a hydroxypropyl content of from 6-10%, a methoxy content of from 20-24%, a phthalyl content of from 21-27%, a molecular weight of about 84,000 daltons known under the trade mark HP50 and available from Shin-Etsu Chemical Co. Ltd., Tokyo, Japan, and having a hydroxypropyl content, a methoxy content, and a phthalyl content of 5-9%, 18-22% and 27-35% respectively, and a molecular weight of 78,000 daltons, known under the trademark HP55 and available from the same supplier.

A preferred coating is HPMCP HP50.

The enteric coating may be carried out in conventional manner, e.g. so that the cores are sprayed with a solution of the enteric-coating. The term "core", as used herein, is understood to mean tablets, granules, pellets or MPA or mycophenolate salt drug substance. Attention is drawn to the numerous known methods of coating employed in the art, e.g. spray coating in a fluidized bed, e.g. by the known methods using apparatus available from Aeromatic, Glatt, Wurster or Hüttlin, in a perforated pan by the Accela Cota method, or to the submerged sword coating method. The additives commonly used in confectioning are employed in such methods.

Suitable solvents for the enteric-coating are for example organic solvents, e.g. an alcohol such as ethanol or a mixture of alcohols, e.g. ethanol and isopropanol, a ketone such as acetone, halogenated hydrocarbons such as CH₂Cl₂ or mixtures of such solvents, e.g. ethanol/acetone, e.g. 1:1 to 10:1, wherein the ethanol part may contain up to 5% isopropanol.

If desirable a softener such as di-n-butylphthalate or triacetin may be added to such a solution, e.g. in a ratio of coating material to softener of from 1: 0.05 to 0.3.

According to the invention, it has been found that it is advantageous to employ MPA or mycophenolate salt in its anhydrous form. Difficulties encountered in the coating of tablets comprising MPA or mycophenolate salt in anhydrous form using an aqueous solvent may include hydrate formation, e.g. various degrees of hydration, and the formation of polymorphs of the drug substance.

In order to meet these and related difficulties, it has now surprisingly been found that a non-aqueous coating, e.g. an organic coating, may be used.

According to the invention, enteric coated tablets comprising MPA or mycophenolate salt in their anhydrous form are obtained using an organic coating by optimization of the amount of coating material and/or optimization of the spraying conditions during the coating process. The absolute amount of the coating material and the spraying conditions may be selected and used having regard to the particular desired properties of the tablet by routine experimentation.

Accordingly, in another embodiment the present invention provides a process for the preparation of an enteric coated tablet comprising MPA or mycophenolate salt in anhydrous form, which process comprises dissolving/dispersing the coating material, e.g. a hydroxypropyl methylcellulose phthalates, e.g. HPMCP HP50, and optionally pigments, e.g. iron oxide, indigotine, e.g. indigotine lake, and/or titanium dioxide, in an organic solvent or a mixture of organic solvents, e.g. ethanol/acetone (w/w), and spraying the solution/dispersion onto the tablets.

In yet a further aspect the present invention provides a method of avoiding hydrate formation comprising dissolving/dispersing the organic coating material in an organic solvent or a mixture of organic solvents, e.g. as already disclosed above, and spraying the solution/dispersion onto the tablets.

Preferably the coating material may be used in an amount of from 5 to 20 % by weight, e.g. 10 to 15% by weight, preferably 10 % by weight based on the total weight of the film-coated tablet.

Desirably, the organic solvent, e.g. ethanol, is substantially anhydrous, containing e.g. less than 15%, more preferably less than 10%, most preferably less than 5% water. Suitable as ethanol may be ethanol 94% (w/w) or absolute ethanol.

A fluidized bed coater or a perforated pan coater may be used for coating.

Conveniently the cores are treated at room temperature or warmed up to 40°C e.g. by means of warm air of 40° up to 70°C, before spraying. To avoid a sticking of the cores the spray procedure is preferably interrupted at certain time intervals and the cores then warmed up again. It is, however, also possible to proceed without interruption of the spray procedure, e.g. by automatic regulation of the spray amount taking into account the temperature of exhaust air and/or cores.

The spray pressure may vary within wide ranges, in general satisfactory results are obtained with a spray pressure of from about 1 to 70 bar, e.g. of from 20 to 60 bar, preferably of from 40 to 50 bar, e.g for an airless spray system.

The invention provides in another of its aspects a process of making tablets as hereinabove described. Such tablets may be produced by
(i) mixing the mycophenolic acid or mycophenolate salt and pharmaceutically acceptable additives,
(ii) subjecting a mixture obtained in step (i) to granulation
(iii) compressing the granulates obtained in step (ii) and pharmaceutically acceptable additives to form the tablet,
and (iv) applying enteric coating of the mycophenolic acid or mycophenolate salt, and/or granulate, and/or tablet, as defined in claim 1. The coating procedure in step (iv) may be performed according to a process as specified above.

The granulation step (ii) may be a wet-granulation, e.g. spray granulation or high shear mixing methods, melt granulation or dry granulation, e.g. roller compaction.

When MPA and mycophenolate salt are used in their anhydrous form all steps as specified above, particularly the granulation step (ii) and the coating procedure are carried out applying non-aqueous solvents only, e.g. organic solvents, e.g. as specified above. Preferably the organic solvent, e.g. ethanol, is substantially anhydrous, e.g. as specified above.

Tablets of the invention may also be produced by direct compression of drug substance and additives comprising step (i) and step (iii), in the absence of step (ii).

In case of unfavourable drug substance properties, e.g. a low bulk density of the drug substance, granulation techniques such as melt granulation, wet granulation or roller compaction may be performed, followed by the compression step. More specifically, in one aspect the present invention provides a process in accordance with claim 1, comprising:
(i) mixing MPA or mycophenolate salt and pharmaceutically acceptable additives, e.g. one or more binders, e.g. polyvinylpyrrollidone, and one or more glidants, e.g. colloidal silicon dioxide, e.g. in a high shear mixer;
(ii) adding a solvent, e.g. ethanol, e.g. 94% (w/w), subjecting the mixture to wetting/kneading, e.g. in a high shear mixer, wet-granulating using, e.g., a rotating impeller, and drying, e.g. in a fluidized bed dryer;
(iii) adding pharmaceutically acceptable additives, e.g. sieved additives, e.g. one or more fillers, e.g. lactose, one or more disintegrants, e.g. crosslinked polyvinylpyrrolidone, one or more lubricants, e.g. magnesium stearate, and mixing, e.g. in a container mixer;
(iv) subjecting the mixture obtained in step (iii) to compression, e.g. in a conventional tabletting machine, e.g. in an EK-0 Korsch eccentric tabletting machine or a rotary tabletting machine, preferably a rotary machine, e.g. at a compression greater than 5 kN; and
(v) applying enteric coating to the mycophenolic acid or mycophenolate salt, and/or granulate and/or tablet.

A tablet obtained according to the present invention containing e.g. 190 mg of mycophenolate salt, and appropriate additives in appropriate quantities is preferably made by a process wherein the compression force used to produce the tablet is of from 15 to 25 kN, preferably 20 kN. Appropriate additives in appropriate quantities for this active agent may be 45 mg lactose, 6.6 mg anhydrous colloidal silica, 3.25 mg magnesium stearate, 20 mg PVP, 10.25 mg maize starch, and 32.5 mg crosslinked PVP. For a tablet containing e.g. 385 mg of mycophenolate salt the compression force used to produce the tablet is of from 15 to 35 kN, preferably 20 kN or 30 kN. The particular minimum compression force is dependent on the active agent content in any given formulation and therefore also depends on the amount and nature of the additives present.

The minimum compression force may be determined for other formulations using routine experimentation

The tablet cores may vary in shape and be, for example, round, oval, oblong, cylindrical or any other suitable shape. A characteristic of tablets according to the invention is their small size having regard to the amount of MPA or mycophenolate salt contained therein.

In a preferred embodiment the tablets obtained by the compression method described above are round or oval. The edges of the tablets may be bevelled or rounded.

In a particularly preferred embodiment of the invention a tablet is compressed into a round shape of dimensions diameter: height 10.0-10.2 mm : 3.9 mm; or of a tablet having an oval shape of dimensions length : width : height 17.0-17.2 : 6.7-6.9 : 5.9 mm.

The tablets obtained according to the invention comprising e.g. 190 mg of mycophenolate salt, may furthermore have a hardness of from 40 to 140 N, e.g. 60 to 110 N, preferably 90 N. The tablets comprising e.g. 385 mg of mycophenolate salt, may furthermore have a hardness of from 90 to 230 N, e.g. 110 to 210 N, preferably 160 N. Tablet hardness is preferably determined according to the standard test e.g. for example using a Schleuniger 6D tablet testing device.

If desired, tablets prepared according to a process as specified above may be coated in e.g. a film-coating machine, e.g. a perforated pan coater, e.g. according to a coating process as specified above, e.g. with a coating material, comprising e.g. hydroxypropyl methylcellulose phthalates, and pigments dissolved/dispersed in a solvent or a mixture of solvents, e.g. a non-aqueous solvent, e.g. ethanol 94% (w/w)/acetone mixture, to obtain, e.g., a film-coated tablet.

In a particularly preferred embodiment of the invention a coated tablet may have a round shape of dimensions diameter: height of 10.1-10.7 mm : 4.2 mm; or an oval shape of dimensions length : width : height of 17.2-18.0 : 6.9-7.5 : 6.3 mm.

The tablets obtained in accordance with the invention may furthermore be coloured, any coating colourless or coloured, and the tablets or coating marked so as to impart an individual appearance and to make them instantly recognizable. The use of dyes can serve to enhance the appearance as well as to identify the compositions. Dyes suitable for use in pharmacy typically include carotinoids, iron oxides, titanium dioxides, indigotine, e.g. indigotine lake, or chlorophyll. Preferably the tablets of the invention are marked using an imprint code.

Procedures which may be used may be conventional or known in the art or based on such procedures e.g those described in L. Lachman et al. The Theory and Practice of Industrial Pharmacy, 3rd Ed, 1986, H. Sucker et al, Pharmazeutische Technologie, Thieme, 1991, Hagers Handbuch der pharmazeutischen Praxis, 4th Ed. (Springer Verlag, 1971) and Remington's Pharmaceutical Sciences, 13th Ed., (Mack Publ., Co., 1970) or later editions.

The tablets obtained according to the invention are useful as immunosuppressants as indicated by standard tests.

The activity and characteristics of the tablets may be indicated in standard
a) clinical trials, e.g. observing the first acute rejection episodes or treatment failure six months after transplant of kidneys or maintaining a rejection - free state within 6 months after initiation of treatment with the invention. The tablets obtained according to the invention are administered at a dose in the range of 0.5 to 2.0 g/day e.g. 1.5 g/day and decrease the acute rejection rates when administered during the period around transplant surgery, and maintain a rejection-free state in patients who are 3 months or more after transplantation. Thus the tablets obtained according to the invention may be administered during the initial 72 hours after transplantation at dose of 0.5 g administered twice a day in combination with a conventional steroid and cyclosporin, e.g. as NEORAL^{R} for which the cyclosporin dose is the conventional dose e.g. ca 8 ± 3 mg/kg for renal transplants. The steroid dose is to be administered at 2.5 mg/kg for 4 days after transplant, 1 mg/kg thereafter for 1 week, 0.6 mg/kg thereafter for 2 weeks thereafter 0.3 mg/kg for 1 month for prednisone, and in
b) animal trials e.g. observing the kidney allograft reaction in rat. In this test one kidney from a female fisher 344 rat is transplanted onto the renal vessel of a unilaterally (left side) nephrectomized WF recipient rat using an end-to-end anastomosis. Ureteric ananstomosis is also end-to-end. Treatment commences on the day of transplantation and is continued for 14 days. A contralateral nephrectomy is done seven days after transplantation, leaving the recipient relying on the performance of the donor kidney. Survival of the graft recipient is taken as the parameter for a functional graft. Typical doses of the tablets obtained according to the invention are from 1 to 30 mg/kg p.o.

The tablets obtained according to the invention are particularly useful for the following conditions:
a) Treatment or prevention of organ, tissue or cellular allograft or xenograft transplant rejection, e.g. for the treatment of recipients of e.g. heart, lung, combined heart-lung, liver, kidney, bowel, pancreatic, skin, pancreatic islet cell, neural cell or corneal transplant; including treatment or prevention of acute rejection; treatment and prevention of hyperacute rejection, e.g. as associated with xenograft rejection; and treatment or prevention of chronic rejection, e.g. as associated with graft-vessel disease. The tablets obtained according to the invention are also indicated for the treatment or prevention of graft-versus-host disease, such as following bone marrow transplantation.
b) Treatment or prevention of autoimmune diseases, e.g. immune-mediated diseases and inflammatory conditions, in particular inflammatory conditions with an etiology including an immunological component such as arthritis (for example rheumatoid arthritis, arthritis chronica progrediente and arthritis deformans) and rheumatic diseases. Specific immune-mediated disease for which the tablets obtained according to the invention may be employed include, autoimmune hematological disorders, including, but not limited to hemolytic anaemia, aplastic anaemia, pure red cell anaemia and idiopathic thrombocytopenia), systemic lupus erythematosus, polychondritis, sclerodoma, Wegener granulosis, dermatomyositis, polymyositis, chronic active hepatitis, primary bilary cirrhosis, myasthenia gravis, psoriasis, Steven-Johnson syndrome, pemphigus, idiophatic sprue, inflammatory bowel disease (including e.g. ulcerative colitis and Crohn's disease), endocrine ophthalmophathy, Graves disease, sarcoidosis, multiple sclerosis, juvenile diabetes (diabetes mellitus type I), non-infectious uveitis (anterior and posterior), keratoconjunctivitis sicca and vernal keratoconjunctivitis, interstitial lung fibrosis, psoriatic arthritis, vasculitis, glomerulonephritides (with and without nephrotic syndrome, e.g. including idiophatic nephrotic syndrome or minimal change nephropathy) and juvenile dermatomyositis.

Appropriate dosage will of course vary, e.g. depending on the condition to be treated (for example the disease type or the nature of resistance), the MPA salt used, the effect desired and the mode of administration.

In general however satisfactory results are obtained on administration e.g. orally at dosages on the order of from 1 to 30 mg salt per kg animal body weight per day, administered once or in divided doses up to 4 times per day. Suitable daily dosages for patients are thus in the order of 200 mg to 3 g p.o. salt e.g. from 50 to 100% that of mycophenolate mofetil. For the preferred mono sodium salt the dosage of the salt is about two thirds that of mycophenolate mofetil.

The bioavailability characteristics may be determined in conventional manner, e.g. by oral administration to beagle dogs. Dosages are typically 50 mg salt/animal e.g. ca 3-5 mg salt/kg animal body weight. Dogs are adult (ca. 10 kg, e.g. 6-14 kg) and fasted. Three hours after administration ca. 200 g food is administered. Blood samples are taken from the cephalic vein, before administration and 10, 30, and 45 minutes, 1, 1.5, 2, 3, 4, 6, 8, 12, and 24 hours, after administration. Plasma levels of free MPA are determined by HPLC analysis (with UV detection).

The tablets obtained according to the invention comprising a therapeutically effective amount of mycophenolic acid or mycophenolate salt may be administered as the sole active ingredient or with another immunosuppressant e.g. together with simultaneous or separate administration of other immunosuppressants, for example, in immunosuppressive applications such as prevention or treatment of graft vs. host disease, transplant rejection, or immune-mediated diseases. For example, the tablets obtained according to the invention may be used in combination with cyclosporins or ascomycins, or their immunosuppressive analogs, e.g. cyclosporin A, FK-506 (tacrolimus), etc., rapamycin or a derivative thereof, e.g. 40-O-(2-hydroxyethyl)-rapamycin, a derivative as disclosed e.g. in WO 95/14023 and 99/15530, e.g. ABT578, or rapalogs as disclosed e.g. in WO 98/02441 and WO01/14387, e.g. AP23573; a lymphocyte homing agent, e.g. FTY720 (2-amino-2-[2-(4-octylphenyl) ethyl]propane-1,3-diol in free form or in a pharmaceutically salt form, e.g.the hydrochloride), corticosteroids; cyclophosphamide; azathioprine; methotrexate; brequinar; leflunomide; mizoribine; deoxyspergualin; or immunosuppressive monoclonal antibodies, e.g., monoclonal antibodies to leukocyte receptors, e.g. MHC, CD2, CD3, CD4, CD7, CD25, CD28, CTLA4, B7, CD40, CD45, or CD58 or to their ligands; or other immunomodulatory compounds. A preferred combination comprises a tablet obtained according to the invention and rapamycin or a derivative thereof, e.g. as indicated above, e.g. 40-O-(2-hydroxyethyl)-rapamycin, and/or a lymphocyte homing agent, e.g FTY720.

When the tablets obtained according to the invention are co-administered with such other immunosuppressants the dosages of the other immunosuppressants may be reduced e.g. to one-half to one-third their dosages when used alone.

Representative doses for ciclosporin to be used are e.g. 1 to 10, e.g. 1 to 2 mg/kg/day. The following Examples serve to illustrate the invention.

| | **Example 1** | **Example 2** |
|---|---|---|
| *Tablet content* | *mg (% of core; % coated tablet)* | *mg* |
| Mycophenolate sodium | 192.4¹ (62.1; 54.2) | 384.8² |
| Lactose, anhydr. (1.1) | 45 (14.5; 12.7) | 90 |
| Crospovidone® (1.2) | 32.5 (10.5; 9.2) | 65 |
| Povidone® K30 (1.3) | 20 (6.5; 5.6) | 40 |
| Maize starch (1.2) | 10.25 (3.3; 2.9) | 20.5 |
| Colloidal silicon dioxide, anhydr. (1.4) | 6.6 (2.1; 1.9) | 13.2 |
| Magnesium stearate (1.5) | 3.25 (1; 0.9) | 6.5 |
| Total (core) | 310 (100; 87.4) | 620 |

| *Enteric coating* | | |
|---|---|---|
| HPMCP HP50 | 42 (-; 11.8) | 65 |
| Iron oxide yellow | 0.078 | 0.167 |
| Iron oxide red | - | 0.167 |
| Titanium dioxide | 2.883 (-;0.8) | 4.666 |
| Indigotine lake | 0.039 | - |
| *Total (coating)* | *45 (-;12.6)* | *70* |
| *Total coated tablet* | *355 (145*/*100)* | *690* |
| Ethanol 94% (w/w), den.³ (Granulation and coating liquid) | | |
| Acetone³ (Coating liquid) | | |
| ¹ Equivalent to 180 mg mycophenolic acid | | |
| ² Equivalent to 360 mg mycophenolic acid | | |
| ³ Lost during the film coat drying stage | | |

### Method

The tablet ingredients mycophenolate sodium, Povidone® K30, silica, colloidal anhydrous are
(i) mixed;
(ii) wet-granulated using ethanol 94% (w/w);
(iii) mixed with lactose anhydrous, maize starch, Crospovidone®, and magnesium stearate; and compressed to tablets preferably 20 kN.
(iv) The tablets are coated in a perforated pan coater with a solution of the coating ingredients in ethanol (with 5% isopropanol)/acetone. The tablets meet the enteric coating test described herein and do not disintegrate within 2 hours in artificial gastric juices (pH 1, HCl). The compositions are stable, e.g. for 2 years with a disintegration of less than 5% mycophenolic acid content at room temperature.

By following above procedure, following tablets (coated and uncoated) may be obtained:

### Example 3

| *Tablet content* | *mg* |
|---|---|
| Mycophenolate sodium | 342^{*} |
| Lactose, anhydr. (1.1) | 80 |
| Crospovidone^{®} (1.2) | 57.80 |
| Povidone^{®} K30 (1.3) | 35.50 |
| Maize starch (1.2) | 18.20 |
| Colloidal silicon dioxide, anhydr. (1.4) | 11.70 |
| Magnesium stearate (1.5) | 5.80 |
| Total (core) | 551 |

| ***Enteric coating*** | |
|---|---|
| HPMCP HP50 | 60 |
| *Total tablet* | *611* |
| ^{*} equivalent to 320 mg MPA | |

### Example 4

A granulate is manufactured using the organic wet granulation process, ethanol abs. being used as granulation liquid.

| | |
|---|---|
| Mycophenolate sodium | 192.3* |
| Lactose 200 mesh (1.1) | 147.7 |
| Crosscarmellose (1.2) | 100.0 |
| Povidone^{®} K30 (1.3) | 35. 0 |
| Colloidal silicon dioxide, anhydr. (1.4) | 20.0 |
| Magnesium stearate | 5.0 |
| Total | 500 |

This granulate may be further mixed with additives and compressed into tablets.

### Enteric coating

| | |
|---|---|
| HPMCP HP50 | 60 |
| Total Tablet | 560 |
| * equivalent to 190 mg MPA | |

## Claims

1. A process for the preparation of an enteric coated tablet comprising a pharmacologically effective amount of mycophenolic acid or mycophenolate salt, wherein the mycophenolic acid or mycophenolate salt is present in an amount of from 20% to 80% by weight based on the total weight of the solid dosage form including the enteric coating, wherein the mycophenolic acid or mycophenolate salt is present in substantially anhydrous form, which process comprises
(i) mixing the mycophenolic acid or mycophenolate salt and pharmaceutically acceptable additives,
(ii) subjecting a mixture obtained in step (i) to granulation,
(iii) compressing the granulates obtained in step (ii) and pharmaceutically acceptable additives to form the tablet, and
(iv) applying enteric coating to the mycophenolic acid or mycophenolate salt and/or to the granulates obtained in step (ii) and/or to the tablet obtained in step (iii), wherein step (ii) and (iv) are carried out applying non-aqueous solvents only,
step (ii) being optional.

2. The process according to claim 1, wherein the tablet contains mycophenolate sodium salt in crystalline form.

3. The process according to claim 1, wherein the tablet contains mycophenolate crystalline mono sodium salt.

4. The process according to any preceding claim, wherein the mycophenolic acid or mycophenolate salt is present in an amount of from 45% to 80% by weight based on the total weight of the solid dosage form including the enteric coating.

5. The process according to any preceding claim, wherein the mycophenolic acid or mycophenolate salt is present in an amount of from 50% to 65% by weight based on the total weight of the solid dosage form including the enteric coating.

## Patentansprüche

1. Verfahren zur Herstellung einer magensaftresistent beschichteten Tablette, die eine pharmakologisch wirksame Menge von Mycophenolsäure oder Mycophenolatsalz umfasst, wobei die Mycophenolsäure bzw. das Mycophenolatsalz in einer Menge von 20 Gew.-% bis 80 Gew.-%, bezogen auf das Gesamtgewicht der festen Dosierungsform einschließlich der magensaftresistenten Beschichtung, vorliegt, wobei die Mycophenolsäure bzw. das Mycophenolatsalz in einer im Wesentlichen wasserfreien Form vorliegt, wobei das Verfahren Folgendes umfasst:
(i) das Mischen der Mycophenolsäure bzw. des Mycophenolatsalzes mit pharmazeutisch unbedenklichen Zusatzstoffen,
(ii) das Granulieren einer in Schritt (i) erhaltenen Mischung,
(iii) das Verpressen des in Schritt (ii) erhaltenen Granulats und pharmazeutisch unbedenklicher Zusatzstoffe unter Bildung der Tablette und
(iv) das Aufbringen einer magensaftresistenten Beschichtung auf die Mycophenolsäure bzw. das Mycophenolatsalz und/oder das in Schritt (ii) erhaltene Granulat und/oder die in Schritt (iii) erhaltene Tablette,
wobei Schritt (ii) und (iv) unter Anwendung von ausschließlich nicht wässrigen Lösungsmitteln durchgeführt werden,
wobei Schritt (ii) fakultativ ist.

2. Verfahren nach Anspruch 1, wobei die Tablette Mycophenolat-Natriumsalz in kristalliner Form enthält.

3. Verfahren nach Anspruch 1, wobei die Tablette kristallines Mycophenolat-Mononatriumsalz enthält.

4. Verfahren nach einem der vorhergehenden Ansprüche, wobei die Mycophenolsäure bzw. das Mycophenolatsalz in einer Menge von 45 Gew.-% bis 80 Gew.-%, bezogen auf das Gesamtgewicht der festen Dosierungsform einschließlich der magensaftresistenten Beschichtung, vorliegt.

5. Verfahren nach einem der vorhergehenden Ansprüche, wobei die Mycophenolsäure bzw. das Mycophenolatsalz in einer Menge von 50 Gew.-% bis 65 Gew.-%, bezogen auf das Gesamtgewicht der festen Dosierungsform einschließlich der magensaftresistenten Beschichtung, vorliegt.

## Revendications

1. Procédé de préparation d'un comprimé à enrobage entérique, comprenant une quantité efficace sur le plan pharmacologique d'acide mycophénolique ou de sel de mycophénolate, où l'acide mycophénolique ou le sel de mycophénolate est présent selon une quantité allant de 20% à 80% en poids sur la base du poids total de la forme de dosage solide y compris l'enrobage entérique, où l'acide mycophénolique ou le sel de mycophénolate est présent sous une forme sensiblement anhydre, lequel procédé comprend les étapes consistant à
(i) mélanger l'acide mycophénolique ou le sel de mycophénolate et des additifs pharmaceutiquement acceptables,
(ii) soumettre un mélange obtenu dans l'étape (i) à une granulation,
(iii) comprimer les granulés obtenus dans l'étape (ii) et les additifs pharmaceutiquement acceptables afin de former le comprimé, et
(iv) appliquer un enrobage entérique à l'acide mycophénolique ou au sel de mycophénolate et/ou aux granulés obtenus dans l'étape (ii) et/ou au comprimé obtenu dans l'étape (iii), où l'étape (ii) et (iv) sont effectuées en n'appliquant que des solvants non aqueux, l'étape (ii) étant facultative.

2. Procédé selon la revendication 1, dans lequel le comprimé contient un sel de mycophénolate de sodium sous forme cristalline.

3. Procédé selon la revendication 1, dans lequel le comprimé contient un sel cristallin de mycophénolate monosodique.

4. Procédé selon l'une quelconque des revendications précédentes, dans lequel l'acide mycophénolique ou le sel de mycophénolate est présent selon une quantité allant de 45% à 80% en poids sur la base du poids total de la forme de dosage solide y compris l'enrobage entérique.

5. Procédé selon l'une quelconque des revendications précédentes, dans lequel l'acide mycophénolique ou le sel de mycophénolate est présent selon une quantité allant de 50% à 65% en poids sur la base du poids total de la forme de dosage solide y compris l'enrobage entérique.
